# NEW EUROPEAN PATENT SPECIFICATION

(11) **EP 0 735 848 B2**
(45) Date of publication and mention of the opposition decision: **03.12.2003**
(45) Mention of the grant of the patent: 15.03.2000
(21) Application number: 94905418.3
(22) Date of filing: 17.12.1993
(51) Int. Cl.: A61F 13/15

(54) **TAMPON**
TAMPON
TAMPON HYGIENIQUE

(30) Priority: 30.12.1992 US 998699; 30.12.1992 US 998700
(43) Date of publication of application: 09.10.1996
(60) Divisional application: 99110988.5
(73) Proprietor: TAMBRANDS, INC., White Plains, New York 10604 (US)
(72) Inventor: WATT, William, Springfield, MA 01119 (US); CAMPION, Terese, A., Enfield, CT 06082 (US); CHILD, William, M., Monson, MA 01057 (US); TARR, Warren, Turners Falls, MA 01376 (US)
(74) Representative: Kremer, Véronique
(86) International application number: US9312295
(87) International publication number: WO94015564

(56) References cited:
- WO-A-90/07314
- DE-C- 945 947
- GB-A- 2 225 949
- GB-A- 3 373 848
- US-A- 2 123 750
- US-A- 2 301 868
- US-A- 2 499 414
- US-A- 3 738 364
- US-A- 3 854 481
- US-A- 4 327 728
- US-A- 5 004 467

## Description

### Background of the Invention

The present invention relates to tampons.

Tampons for use in absorption of menstrual flow have been inserted using an applicator, as is well known. Other tampons, known as "digital" tampons, have been inserted manually by the user, without the need for an applicator, thereby eliminating waste and providing a smaller, more compact product.

Most digital tampons are produced from rolled or spirally wound pledgets. Generally, the withdrawal cord of the tampon is rolled into the pledget and extends from the center of the withdrawal end. When the withdrawal cord is attached in this manner, the user can "flare" the withdrawal end of the tampon prior to insertion, to provide a well for the user's finger, by holding the tampon and pulling the cord against the periphery of the withdrawal end.

When the withdrawal cord is rolled into the pledget, however, the tampon may "telescope" when withdrawn, i.e., the central windings may be pulled out of the wound tampon. Accordingly, an alternate method of cord attachment was developed, in which the withdrawal cord is punched through the pledget, e.g., as disclosed in U.S. 5,004,467. These tampons, while eliminating the problem of telescoping, typically cannot be flared by the user, making insertion more difficult.

Rolled tampons may be formed by providing a section of specific length of an absorbent material, e.g. a nonwoven web, having a width corresponding approximately to the length of the tampon, winding or rolling the section upon itself to form a pledget, and compressing the pledget radially.

Such a tampon made of a rolled non woven fabric is for example described in US 3,854,481. More particularly the tampon described in US 3,854 481 have an insertion end and a rolled body portion, the insertion end being compressed to a density less than the density of the rolled body portion.

Tampons are often provided with an overwrap, i.e. an cuter covering of a liquid permeable material, typically a thermoplastic film, to improve the lubricity of the tampon, reducing insertion and withdrawal forces, and to prevent fibers of the nonwoven from being detached ("fiber fluff-off") during insertion and withdrawal.

Conventional methods of overwrapping tampons, e.g., that disclosed in U.S. 5,004,467, leave excess, baggy overwrap at the tampon base after compression of the pledget, increasing the difficulty of insertion, and reducing the aesthetic qualities of the product.

A process for providing a tampon with an overwrap is disclosed in U.S. 5,004,467. According to this process, the pledget is enclosed in a cylindrical envelope of overwrap material, which is open at the insertion end. The margin of material at the open end is folded over a portion of the head of the pledget, leaving an open, uncovered area in the center of the insertion end of the tampon.

One problem which may occur with overwrapped tampons is peeling back of the overwrap from the surface of the absorbent material during insertion or when the tampon is removed after use ("peel back"). This may cause discomfort and disturb the user, and may even result in part or all of the overwrap tearing off.

### Summary of the Invention

The present invention provides an improved tampon, particularly suited for use as a digital tampon.

In one aspect, the invention features a digital tampon which includes an elongated pledget formed of a multi-layer roll of absorbent material that has been radially compressed, and a withdrawal cord attached to the pledget. The pledget has, at one end, an insertion end shaped for insertion into a body cavity, and, at the other end, a withdrawal end shaped to permit the user to apply an axial force thereto for inserting the tampon. The fiber density of the pledget in the vicinity of the withdrawal end is lower than the average fiber density of the pledget. The reduced fiber density of the withdrawal end allows the user to easily flare the soft withdrawal end, providing a well for digital insertion.

The tampon of the invention preferably also includes an indentation in the compressed pledget, extending axially from the withdrawal end into the interior of the pledget. This indentation further facilitates flaring, provides a finger well for the user even if the user does not flare the withdrawal end, and allows excess overwrap at the withdrawal end of the tampon to be tucked in. Preferably, the indentation comprises a substantially hemispherical region nearest the withdrawal end followed by a substantially conical region further from the withdrawal end.

Prior to compression, radially central portions of the absorbent material are displaced at the withdrawal end in an axial direction toward the insertion end,.resulting in increased fiber density in at least some locations along the central region of the tampon. This higher fiber density provides a firmer, denser central core for transferring force between a pushrod (applied to the withdrawal end) and a header (applied to the insertion end) during the head-forming operation in a tampon manufacturing process. This improved transfer of force allows formation of more elongated head shapes which are difficult to form on conventional tampons; e.g., the length of the head can be made at least 75% (and more preferably more than 100%) of the diameter of the pledget. The invention also provides a more stable head. Head formation is accomplished with a lower average fiber density than with conventionally formed pledgets, providing a softer tampon with a greater absorbant efficiency.

The tampon of the invention can thus be made softer than conventional tampons, allowing the tampon to expand more easily and provide higher absorbency. The high absorbency of the tampon, in turn, allows a lower tampon weight for a given absorbency.

The tampon is constructed by rolling a layer of absorbent material. During the above mentioned displacement of central portions of the uncompressed tampon, some central layers of material are displaced to some degree throughout the length of the pledget and project outwardly at the insertion end, to give a rounded shape to the insertion end. The tampon further includes a permeable overwrap material surrounding at least a portion of the pledget.

In another aspect, the invention relates to a method of forming a digital tampon, having an insertion end shaped for insertion into a body cavity, a withdrawal end shaped to permit the user to apply an axial force thereto for inserting the tampon, and having a cord attached thereto for withdrawal from the cavity. The method includes the steps of: (a) rolling a length of compressible absorbent material to form a pledget with one end corresponding to the withdrawal end and another end corresponding to the insertion end, (b) displacing radially central layers of the absorbent material from the withdrawal end of the pledget towards the insertion end, (c) radially compressing the pledget, and (d) forming an indentation in the withdrawal end of the compressed pledget.

In preferred embodiments, the indentation is formed by a pushrod having an elongated, substantially conical member extending from a substantially hemispherical base.

In another aspect, the invention features an improved tampon, the surface of which is completely and securely covered with a porous overwrap material. Coverage of the entire surface with overwrap makes insertion of the tampon more comfortable, and virtually eliminates fiber fluff off. Additionally, the overwrap is attached to the tampon in a manner which substantially reduces the possibility of detachment (peel back) during insertion and/or removal of the tampon. The tampon includes an indentation which, in embodiments in which it is disposed' at the withdrawal cord end, aids in placement of the tampon if it is to be inserted without an applicator. Since the overwrap covers the entire outer surface of the tampon, the tampon can include materials which otherwise would not have adequate integrity for use in a tampon, e.g., materials which would otherwise come out of the rolled tampon.

The tampon includes a compressible, substantially cylindrical pledget, including a rolled length of absorbent material, having a substantially conical depression in one end. A permeable overwrap material, in the farm of a cylindrical envelope, completely surrounds the pledget. The envelope has a bottom end wall which is adjacent the end of the pledget opposite the depression, a tubular wall which surrounds the peripheral surface of the pledget, and a gathered portion which is tucked into the center of the depression, which corresponds to the center of the rolled absorbent. A withdrawal cord either extends from the end having the depression, making this the withdrawal end of the finished tampon, or the opposite end, making the end having the depression the insertion end instead.

In a preferred embodiment, the bottom end wall of the envelope is adjacent the withdrawal end of the tampon, the gathered portion is tucked into a depression in the insertion end, and the depression is filled with a material different from the pledget material, e.g., a material with a different absorbency or expansion capability. Accordingly, the way in which the tampon absorbs fluid and expands can be controlled by the designer by selecting the materials used in the pledget and filler. For example, the tampon could have a soft, conformable outer layer, providing comfort for the user, and an inner layer of higher absorbency which would draw fluid into the core of the tampon.

In this embodiment, the depression may be deep, forming a "cup-shaped" tampon, i.e,. a tampon which, when compressed appears similar to standard tampons but upon absorbing fluid "blooms" to a cup shape, providing a tampon having superior comfort and absorbency.

In another preferred embodiment, the tubular wall of the overwrap envelope includes a plurality of sections, including two substantially semicylindrical sections having overlapping marginal portions extending from the bottom end wall to the gathered portion.

The term "pledget", as used herein, refers to a cylindrical blank including a length of rolled absorbent material, which blank is subsequently compressed to form a finished tampon.

Other features and advantages of the invention will be apparent from the following description of the preferred embodiments and from the claims.

### Brief Description of the Drawings

Fig. 1 is a perspective view of a tampon according to one embodiment of the invention.
Fig. 1a is a cross-sectional view of the tampon of Fig. 1, taken along line 1A-1A.
Fig. 2 is a perspective view of the tampon of Fig. 1 after flaring.
Fig. 2a is a cross-sectional view of Fig. 2, taken along line 2A-2A.
Figs. 3a-3f are a schematic flow diagram of a process for forming a tampon according to one embodiment of the invention.
Fig. 4 is a side plan view of a conical member according to one embodiment of the invention.
Fig. 4a is a top plan view of the conical member of Fig. 4.
Fig. 5 is a side plan view of a push-rod according to one embodiment of the invention.
Fig. 6 is a cross-sectional view of an uncompressed pledget after the processing step shown in Fig. 3b.
Fig. 7 is perspective view of a pledget according to one embodiment of the invention.
Fig. 8 is a cross-sectional view of the pledget shown in Fig. 7, taken along line 2-2.
Fig. 9 is a perspective view of a pledget according to another embodiment of the invention.
Fig. 10 is a cross-sectional view of the pledget of Fig. 9, taken along line 4-4.
Fig. 11 is a cross-sectional view of a pledget according to another embodiment of the invention.
Fig. 12 is a flow diagram showing a process for manufacturing a tampon according to one embodiment of the invention.

### Description of the Preferred Embodiments

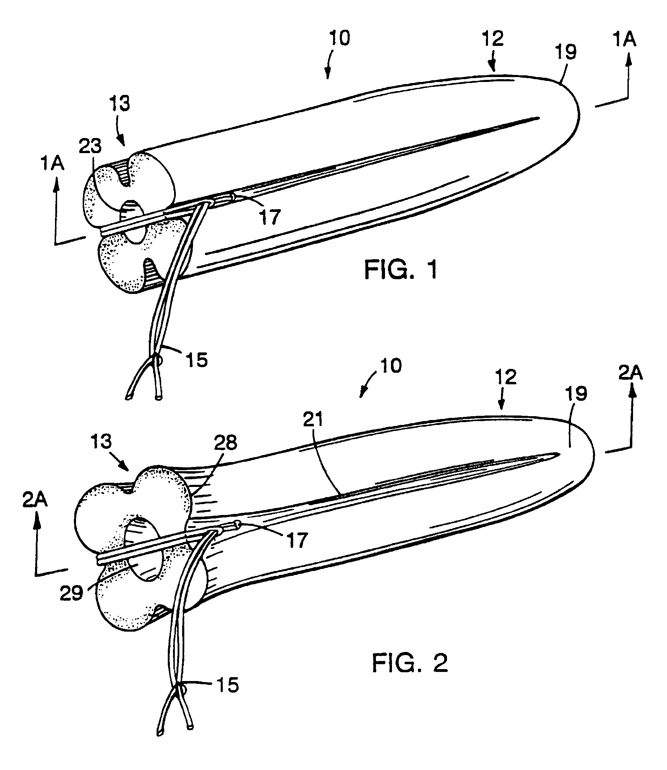

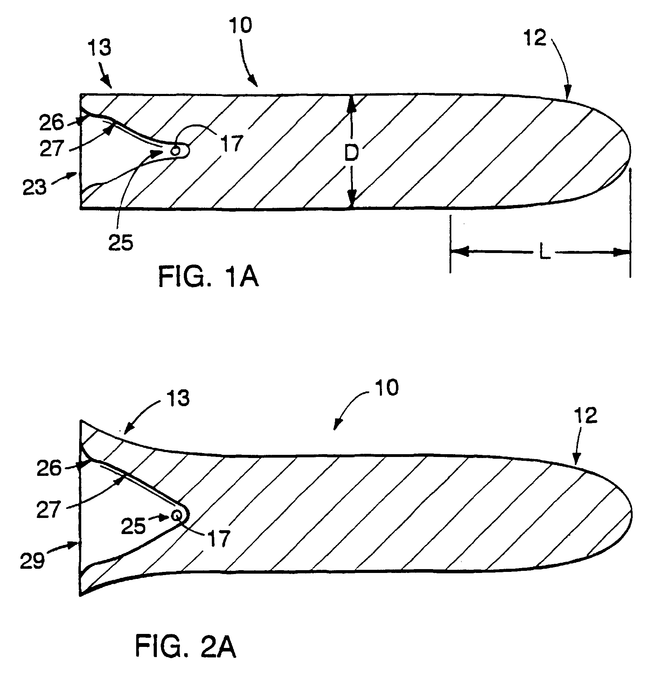

Referring to Figs. 1 and 1a, a tampon 10 is shown in the condition in which it would be sold to a consumer. Tampon 10 includes insertion end 12, withdrawal end 13, and withdrawal cord 15. Withdrawal cord 15 is threaded through aperture 17 which extends transversely through the tampon, through opposing longitudinal compression indentations 21. Withdrawal cord 15 is fastened with a slip knot, which is located in indentation 21, rather than at the base of the tampon, for optimal flarability. Insertion end 12 preferably has a smooth, elongated head 19, with head length L (Fig. 1a) approximately 150% of pledget diameter D. This provides an elliptical or "bullet" shape for optimal ease of insertion. Other head shapes may be used in some aspects of the invention, e.g., conventional domed head shapes (in which head length L is only 50% of diameter D). The tampon also preferably includes longitudinal compression indentations 21.

An axial indentation 23 is defined in the withdrawal end 13 of the tampon. A preferred shape for this indentation is shown in Fig. 1a. As shown, the indentation comprises a substantially conical region 25, terminating at the withdrawal end in a substantially hemispherical region 27. Conical region 25 allows the indentation to extend further into the interior of the tampon than would be possible if the indentation were entirely hemispherical, thereby improving the softness of the withdrawal end. Conical region 25 also allows excess overwrap material at the withdrawal end to be securely tucked in. Hemispherical region 27 provides a finger indent or well which is better dimensioned for a user's finger than if the indentation were entirely conical. Taper region 26, around the circumference of the hemispherical region, results from the outer layers of the rolled pledget being pulled inward by the adjoining inner layers as the inner layers are displaced.

Referring to Figs. 2 and 2a, the tampon of Figs. 1 and 1a is shown after flaring. The absorbent material at the withdrawal end has been spread out by the user, forming a flare 28, and the indentation has expanded to provide a finger indent 29. The user is thus provided with a large, soft area for digital insertion.

Any conventional absorbent material is suitable for use in the tampon of the invention. Preferred absorbent materials are selected from the group consisting of cellulosic fibers, cotton fibers, rayon fibers and blends thereof. The fiber density of the absorbent material is greater in the vicinity of the insertion end than in the vicinity of the withdrawal end, so that the withdrawal end is soft and easy to manipulate, while the insertion end is hard and stable for ease of manufacturing (head forming) and comfortable insertion. This density differential is preferably accomplished by displacing a portion of the absorbent material of the uncompressed pledget from the withdrawal end toward the insertion end.

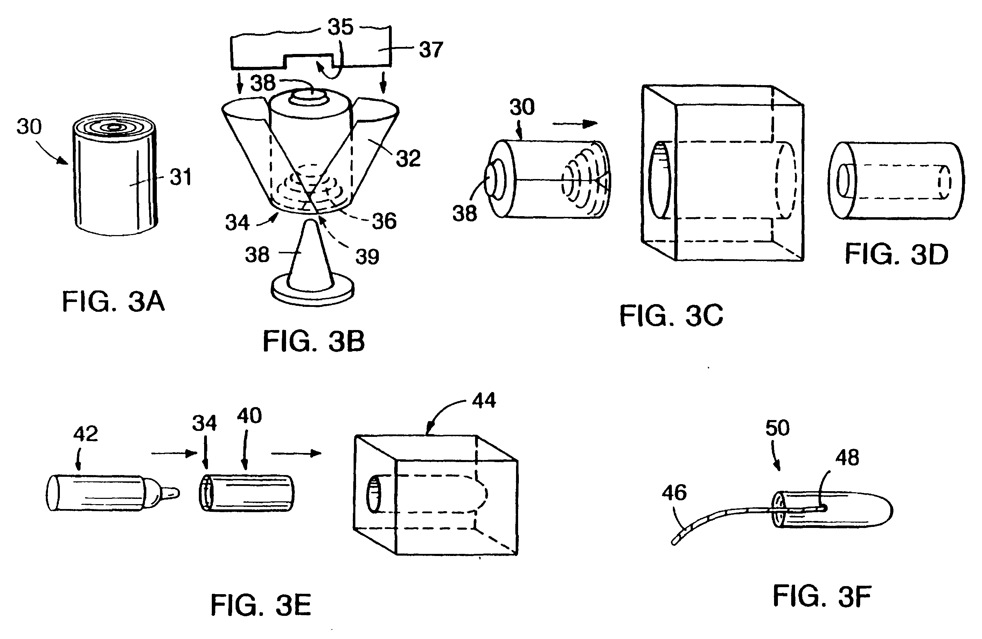

A preferred method of forming a tampon of the invention is shown schematically in Figs. 3a-3f. First, an uncompressed pledget 30 of rolled absorbent material 31 is provided (Fig. 3a). The pledget can be rolled using conventional winding equipment, e.g. those available from Karl Ruggli AG, Fisibach, Switzerland, or the "Falu" made by K. Fassbind-Ludwig & Co., Fulu Machinbau, Wagen Bei Jona, Switzerland.

Next, a sheet of overwrap material 32 is placed adjacent a first end 34 of the pledget, and folded around a portion of the pledget (Fig. 3b), preferably in the form of an envelope as described in U.S. Patent 5,004,467. The overwrap material may be a permeable thermoplastic, e.g., polypropylene, polyethylene, or blends thereof, or may be a fabric or a non-woven. (If a tampon without overwrap is desired, this step may be omitted.)

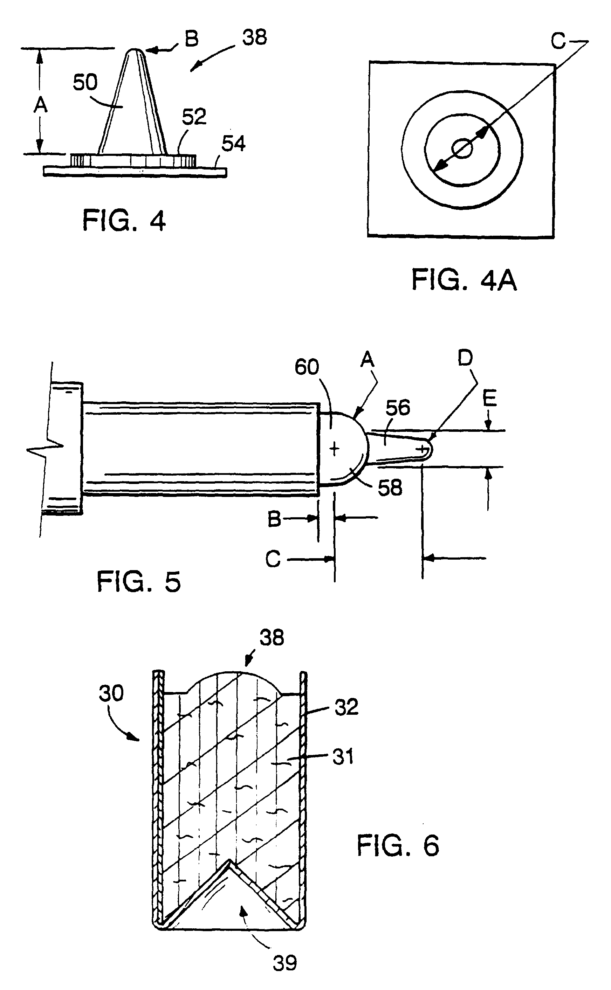

Referring again to Fig. 3b, a portion 36 of the absorbent material at the first end is displaced by pressing the pledget onto a conical die 38, shown in detail in Figs. 4 and 4a, or, alternatively, by pressing the conical die into the pledget. The displacement of material at the first end creates a conical depression 39 in the first end. The pledget may be pressed onto the conical die simultaneous with formation of the overwrap envelope, as shown, or after formation of the envelope. Preferably, at least a portion of the opposite end of the pledget is unrestrained during this step, e.g. by providing a cavity 35 in the ram 37 which urges the pledget against the conical die, as shown, to allow material 38 at the opposite end to be pushed up by the material which is being displaced. More preferably, about one third of the volume which is displaced from the first end is allowed to be pushed up at the opposite end. This pushed up material generally aids in head formation. A cross-sectional view of an uncompressed pledget, subsequent to application of the overwrap and formation of the depression, is shown in Fig. 6.

The pledget is next compressed (Fig. 3c) and heat conditioned (Fig. 3d) in a conventional manner. Compression pressures are well known in the art. Heat conditioning time and temperature will be determined based on head formation requirements, the moisture of the absorbent material used, production speed constraints, whether an overwrap material is used, and, if so, what type of overwrap material. Optimal time and temperature can be readily determined by one skilled in the art.

The compressed pledget 40 is then pressed, using a pushrod 42 (shown in detail in Fig. 5) dimensioned to produce an indentation in the pledget, against a head-forming heated die 44 (Fig. 3e). What was the first end 34 of the uncompressed pledget (the end from which material was displaced) is pressed by the pushrod, while the opposite end contacts the die. If there is excess overwrap material at the first end of the pledget after compression, this step will cause the excess to be tucked into the indentation formed by the pushrod. This head formation step is facilitated by the displacement of the fibers in the withdrawal end towards the insertion end (Fig. 3b). Fiber displacement causes the fiber density to be increased along the central core of the pledget, giving the pushrod a firmer area to push against than it would have in a compressed pledget which was not subjected to this fiber displacement. Thus, force is translated better between the pushrod and the head-forming die, allowing the formation of more acute angles, e.g., formation of bullet shaped heads. Less axial force is needed to achieve a given head elongation, and thus a softer tampon results.

Finally, a withdrawal cord 46 is punched through a transverse aperture 48 in the compressed pledget (Fig. 3f), and secured, e.g., using a slip knot, to form the finished tampon 50.

Steps 3b-3f may be accomplished using a machine commercially available from Hauni Machines, Richmond, VA, under the tradename TAMPOMAT D-500, modified to include the conical die of the invention and to replace the standard Hauni pushrod, which has a flat or concave end, with a pushrod of the invention, e.g., the pushrod shown in Fig. 5, dimensioned to form an indentation in the withdrawal end.

A preferred conical die 38 for use in the displacement step (see Fig. 3b) is shown in Figs. 4 and 4a. Conical die 38 includes conical portion 50, disk 52, and base 54, upon which the disk and conical portion are mounted. Conical portion 50 forms depression 39 shown in Fig. 3b. Disk 52 acts as a guide for positioning the conical die in the Hauni TAMPOMAT machine. If different machinery is used, a different type of guide could be used, as appropriate. Base 54 serves to hold the die in the Hauni machine, and thus, like disk 52, may not be necessary, depending upon the type of production machinery used. Typical dimensions of the die are as follows: A, the height of the conical portion, from about 1,27.10⁻² to 3,17.10⁻² meter (0.5 to 1.25 inch); B, the radius of curvature of the conical portion, about 3.175 10⁻³ meter (0.125 inch); and C, the diameter of the base of the conical portion, from about 2,03.10⁻² to 3,55.10⁻² meter (0.80 to 1.40 inch).

A preferred pushrod 42, for use in the indentation step (see Fig. 3e) is shown in Fig. 5. Pushrod 42 preferably comprises conical portion 56 and hemispherical portion 58, which form, respectively, conical region 25 and hemispherical region 27 in the compressed pledget, as shown in Fig. 1A. Hemispherical portion 58 optionally is mounted on cylindrical base 60. Preferred dimensions of the pushrod are as follows: A, the radius of curvature of the hemispherical portion, from 2,59.10⁻³ to 5,08.10⁻³ meter (0.10 to 0.20 inch); B, the height of the cylindrical base, about 1,27.10⁻³ meter (0.05 inch); C, the height of the pushrod from the base of the hemispherical portion to the base of the hemispherical tip of the conical portion, from 7,62.10⁻³ to 15,24.10⁻³ meter (0.30 to 0.60 inch); D, the radius of curvature of the tip of the conical portion, about 1,02.10⁻³ meter (0.040 inch); and E, the diameter of the conical portion at its base, from 1,90.10⁻³ to 3,81.10⁻³ meter (0.075 to 0.150 inch) (this dimension will typically vary with the diameter of the hemispherical portion). The maximum diameter of the hemispherical portion is limited by the diameter of the compressed pledget, typically about 15 to 40 percent smaller.

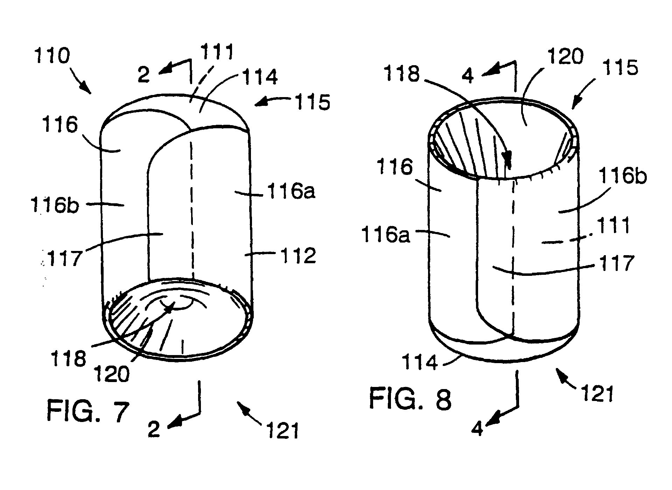

Figs. 7-12 show further embodiments.

Referring to Figs. 7 and 8, overwrapped pledget 110 includes a substantially cylindrical, rolled length of absorbent material 111, completely surrounded by overwrap 112. Overwrap 112 is in the form of an envelope, which includes bottom end wall 114 disposed adjacent insertion end 115, tubular wall 116 surrounding the periphery of the pledget, and gathered portion 118, which is tucked into the center of depression 120 in withdrawal end 121, i.e., into the center of the rolled absorbent material. Depression 120 is preferably about 10 mm to 25 mm deep. The length of the pledget is approximately 40 mm to 55 mm. Tubular wall 116 includes semicylindrical portions 116a and 116b, which overlap in marginal area 117. A withdrawal cord, not shown, is punched through the absorbent material, distally of withdrawal end 121 and passing through marginal area 117, after the pledget is compressed to form a finished tampon.

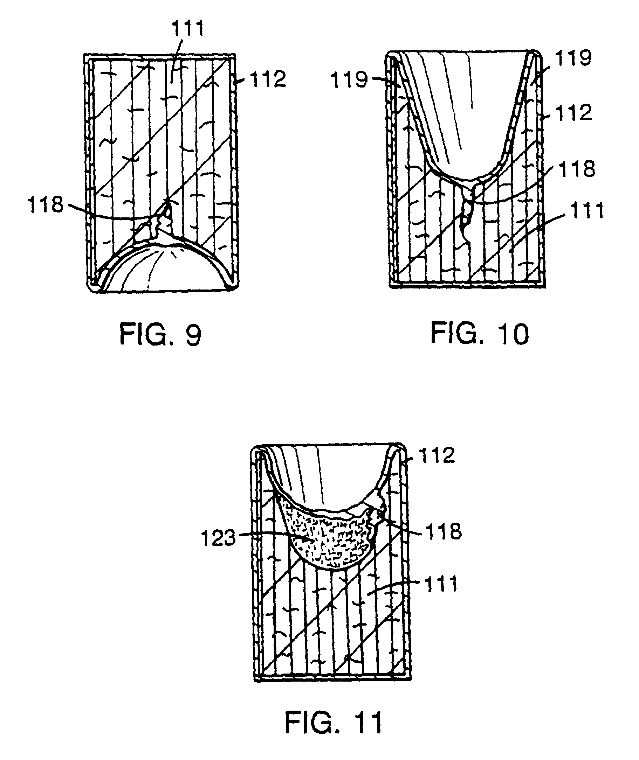

Figs. 9 and 10 show a pledget for a "cup-shaped" tampon, in which depression 120 is in insertion end 115, instead of withdrawal end 121. Accordingly, the open end of the overwrap envelope, which forms the gathered portion 118 is also at the insertion end, and is tucked into the depression formed therein. The walls 119 of the cup-shaped area are preferably from about 3 mm to 5 mm thick. The depression in the insertion end, in this embodiment, is preferably deeper than the depression in the embodiment shown in Fig. 7, more preferably at least 10 mm deep, and most preferably from about 10 mm to 25 mm deep for a pledget having a length of 40 mm to 50 mm, or from about 20% to 65% of the length of a tampon having a different length. This deep depression creates a cup in the center of the pledget, which can advantageously be filled with material 123, as shown in Fig. 11. Material 123 can be selected from a variety of desired materials, e.g., a material with a different absorbency or expansion capability, or rate of absorbency or fluid flow (wicking) profile. The depression is filled, or partially filled, prior to tucking the overwrap in. The filler may itself be in rolled or spiral form, in which case the gathered overwrap is tucked through the centers of both rolls, or the filler may be in solid or powder form, in which case the gathered overwrap is pushed through or past the filler and into the center of the rolled pledget. As in the embodiment shown in Fig. 7, a withdrawal cord, not shown, is punched through the absorbent material, distally of withdrawal end 121 and passing through marginal area 117, after the pledget is compressed to form a finished tampon.

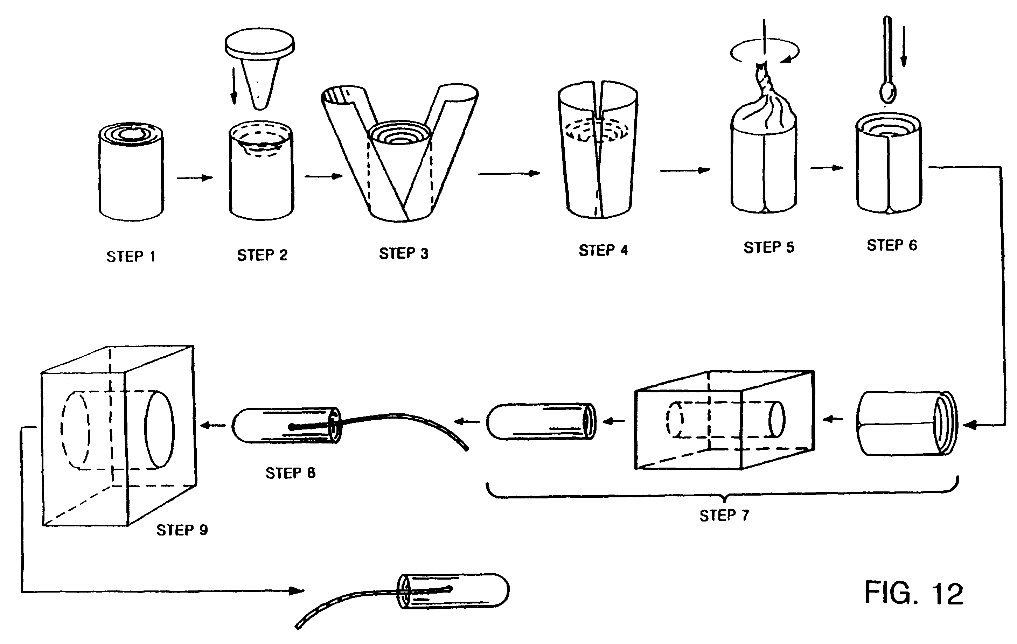

Fig. 12 shows a preferred process for manufacturing an overwrapped tampon having the gathered overwrap tucked into the withdrawal end. First, a rolled pledget is provided (step 1). Then, the center windings of the roll are displaced inward by a suitably shaped die, forming a conical depression in the cord end of the pledget (step 2). It is preferred that the opposite end of the tampon be held by a die or member (not shown) which restricts the corresponding displacement of the roll at that end, so that the material displaced by the conical die is instead compressed. The indentation will be deeper than that shown in Fig. 12 if a "cup-shaped" tampon is desired.

Next, an overwrap blank is placed adjacent the insertion end of the pledget (step 3), and folded into an envelope surrounding the pledget (step 4), e.g., using a folding die as shown in Fig. 4 of U.S. 5,004,467. It is preferred that the overwrap material at the open end of the envelope extend at least about 1.2 cm beyond the end of the tampon. In general, it is preferred that the overwrap at the open end of the envelope extend for a distance equal to at least half the diameter of the rolled pledget. The overwrap material at the open end of the envelope is then gathered and sealed (step 5), e.g. by heat sealing. The material may be gathered manually, or using a machine having a plurality of "fingers" which are brought together around the material. If a non-heatsealable overwrap material is used, the gathered material may be sealed by other means, e.g., adhesive or stitching. The gathered end is pushed into the depression (step 6), e.g., with a pushrod, after which the tampon is compressed (step 7) and, optionally, the compressed tampon may then pass through a conditioning station (step 8), in which it is heated to stabilize the fibers of the absorbent material in a compressed state. Next, a withdrawal cord is punched through an end of the tampon (step 9) which end accordingly becomes the withdrawal end of the finished tampon. As discussed above, the indentation may be at the withdrawal end, in one embodiment, or at the opposite end, in an alternate embodiment; this will be determined by which end the withdrawal cord is punched through. Compression of the pledget, using conventional methods, serves to firmly secure the tucked material in place. Compression is then followed by heat conditioning and formation of a head in the insertion end of the tampon, as is well known.

Any conventional absorbent material is suitable for use in the tampon of the invention. Preferred absorbent materials are selected from the group consisting of cellulosic fibers, cotton fibers, rayon fibers and blends thereof. Most preferred are blends of cotton and rayon fibers.

The tampons of the invention may be manufactured using commercially available tampon winding machines made by Karl Ruggli AG, Fisibach, Switzerland, or, the "Falu" made by K. Fassbind-Ludwig & Co., Fulu Machinenbau, Wagen bei Jona, Switzerland, to wind the pledget, and a machine commercially available from Hauni Machine, Richmond, VA, under the tradename TAMPOMAT to perform the other processing steps.

The overwrap may be a permeable thermoplastic, e.g., polypropylene, polyethylene, or blends thereof, or may be a fabric or a non-woven. Heat sealable materials are generally preferred, as heat sealing is a preferred method of sealing the gathered material. Preferably, the overwrap material is a bicomponent polypropylene/polyethylene nonwoven, having a thickness of from about 0.05 mm to 0.30 mm, and a basis weight of from about 7 g/m² to 25 g/m², more preferably about 10 g/m² to 14 g/m².

While preferred embodiments have been described above, other variations and modifications are within the scope of the following claims. E.g., the depression in one end of the uncompressed pledget can have a variety of shapes other than conical.

## Claims

1. A tampon (10) for digital insertion, the tampon comprising:
- an elongated pledget formed of absorbent material that has been radially compressed,
- a withdrawal cord (15) attached to the pledget,
- an insertion end (12) at one end of the pledget, the insertion end being shaped for insertion into a body cavity, and
- a withdrawal end (13) at the other end of the pledget, the withdrawal end being shaped to permit the user to apply an axial force thereto for inserting the tampon,
- the fiber density in the vicinity of the withdrawal end (13) being lower than the average fiber density of the pledget,
- the fiber density in the vicinity of the insertion end (12) being greater than in the vicinity of the withdrawal end (13) and the length L of the head being at least 75 % of the diameter D of the pledget, wherein the tampon comprises a multi-layer roll of absorbent material that is radially compressed, wherein, prior to radial compression, radially central layers of the absorbent material at the withdrawal end are displaced axially toward the insertion end.

2. The tampon of claim 1 wherein the withdrawal end has an indentation (23) extending axially from the withdrawal end (15) into the interior of the pledget, and the withdrawal end is susceptible of being flared to provide a well for a user's finger.

3. The tampon of claim 1 or 2 wherein the withdrawal cord (15) is attached by means of passing it through a hole extending transversely through the pledget, and the hole for the withdrawal cord is nearer the withdrawal end than the insertion end.

4. The tampon of claim 1, 2, or 3 wherein length L of the head is greater than the diameter D of the pledget.

5. The tampon of claim 2 further comprising a permeable overwrap material (32)(112) applied to at least a portion of the pledget prior to its being radially compressed, wherein the permeable overwrap material covers the withdrawal end of the pledget, and wherein excess overwrap material at the withdrawal end is tucked into the indentation.

6. The tampon according to claim 5 wherein the permeable overwrap material (112) is in the form of an envelope, completely surrounding the pledget wherein said envelope has a bottom end wall (114) which is adjacent the insertion end (115) of the pledget, a tubular wall (116) which surrounds the peripheral surface of the pledget and a gathered portion (118) which is tucked into the indentation (120) extending axially from the withdrawal end into the interior of the pledget.

7. The tampon of any preceding claim wherein, prior to radial compression, at least some of the displaced layers of absorbent material are displaced to some degree throughout the length of the pledget and project outwardly at the insertion end, to give a rounded shape to the insertion end.

8. The tampon of any preceding claim wherein the axial displacement of central portions of the absorbent material has the effect of increasing fiber density in at least some locations along the axially-extending central region of the tampon as compared to the density that would have resulted in that region had there been no such displacement.

## Patentansprüche

1. Ein Tampon (10) zur Fingereinführung, wobei der Tampon umfaßt:
- eine längliche Kompresse aus einem absorbierenden Material, das radial zusammendgedrückt ist,
- ein Rückholfaden (15), der an der Kompresse angebracht ist,
- ein Einführende (12) an einem Ende der Kompresse, wobei das Einführungsende zur Einführung in einen Körperhohlraum geformt ist, und
- ein Rückholende (13) an dem anderen Ende der Kompresse, wobei das Rückholende so geformt ist, daß es der Benutzerin gestattet, zur Einführung des Tampons eine axiale Kraft darauf auszuüben,
- wobei die Faserdichte in der Nähe des Rückholendes (13) geringer als die mittlere Faserdichte der Kompresse ist,
- wobei die Faserdichte in der Nähe des Einführendes (12) größer als in der Nähe des Rückholendes (13) ist, und die Länge L des Kopfes wenigstens 75% des Durchmessers D der Kompresse beträgt,
- wobei der Tampon eine mehrschichtige Rolle von absorbierendem Material umfaßt, die radial zusammengedrückt ist, wobei vor der radialen Zusammendrückung radial zentral liegende Schichten des absorbierenden Materials an dem Rückholende axial zum Einführende hin verschoben sind.

2. Tampon nach Anspruch 1, in welchem das Rückholende eine Vertiefung (23) aufweist, die sich axial von dem Rückholende (15) in das Innere der Kompresse erstreckt, und das Rückholende gekelcht werden kann, um ein Loch für den Finger einer Benutzerin zu schaffen.

3. Tampon nach Anspruch 1 oder 2, in welchen der Rückholfaden (15) angebracht ist, indem dieser durch ein sich quer durch die Kompresse erstreckendes Loch hindurch gefädelt ist und das Loch für den Rückholfaden näher am Rückholende als am Einführende ist.

4. Tampon nach Anspruch 1, 2 oder 3, in welchem die Länge L des Kopfes größer als der Durchmesser D der Kompresse ist.

5. Tampon nach Anspruch 2 ferner mit einem durchlässigen Umhüllungsmaterial (32)(112), das wenigstens auf einem Bereich der Kompresse aufgebracht ist, bevor sie radial zusammengepreßt wird, bei welchem das durchlässige Umhüllungsmaterial das Rückholende der Kompresse überdeckt und bei welchem überschüssiges Umhüllungsmaterial am Rückholende in die Vertiefung gesteckt ist.

6. Tampon nach Anspruch 5, in welchem das durchlässige Umhüllungsmaterial ( 112) die Form eines Umschlags aufweist, der die Kompresse vollständig umgibt, wobei der Umschlag aufweist eine bodenseitige Stirnwand (114), welche an das Einführende (115) der Kompresse angrenzt, eine rohrförmige Wand (116), welche die Umfangsfläche der Kompresse umgibt, und einen gerafften Bereich (118), welcher in die sich axial von dem Rückholende in das Innere der Kompresse erstreckende Vertiefung (120) eingesteckt ist.

7. Tampon nach jedem vorangegangenen Anspruch, in welchem vor der radialen Zusammendrückung wenigstens einige der verschobenen Schichten aus absorbierendem Material um ein gewisses Maß über die Länge der Kompresse hinaus verschoben sind und an dem Einführende nach außen vorstehen, um dem Einführende eine abgerundete Form zu verleihen.

8. Tampon nach jedem vorangegangenen Anspruch, in welchem die axiale Verschiebung von zentralen Bereichen aus absorbierendem Material die Wirkung einer Erhöhung der Faserdichte an wenigstens einigen Stellen entlang des sich axial erstreckenden Zentralbereichs des Tampons hat, verglichen mit der Dichte, die sich in diesem Bereich ergeben würde, wenn eine solche Verschiebung nicht vorläge.

## Revendications

1. Tampon (10) pour insertion digitale, le tampon comprenant:
- une compresse allongée, formée de matériau absorbant qui a été soumise à une compression radiale,
- une cordelette de retrait (15) attachée à la compresse,
- une extrémité d'insertion (12) à une extrémité de la compresse, l'extrémit d'insertion étant configurée de façon à être insérée dans une cavité corporelle, et
- une extrémité de retrait (13) à l'autre extrémité de la compresse, l'extrémité de retrait étant configurée de façon à permettre à l'utilisatrice d'y appliquer une force axiale pur insérer le tampon,
- la densité des fibres au voisinage de l'extrémité de retrait (13) étant inférieure à la densité moyenne de fibres de la compresse,
- la densité des fibres au voisinage de l'extrémité d'insertion (12) étant plus grande qu'au voisinage de l'extrémité de retrait (13), et la longueur L de la tête étant égale à au moins 75% du diamètre D de la compresse, le dit tampon comprent
- un rouleau multi-couche de matériau absorbant qui est soumis à une compression radiale, dans lequel, avant la compression radiale, les couches centrales dans la direction radiale du matériau absorbant, au niveau de l'extrémité de retrait, sont déplacées dans la direction axiale vers l'extrémité d'insertion.

2. Tampon selon la revendication 1, dans lequel l'extrémité de retrait présente un évidement (23) s'étendant dans la direction axiale depuis l'extrémité de retrait (15) vers l'intérieur de la compresse, et l'extrémité de retrait est susceptible de s'évaser afin de former un puits pour un doigt d'une utilisatrice.

3. Tampon selon la revendication 1 ou 2, dans lequel la cordelette de retrait (15) est attachée par le fait de passer dans un trou s'étendant transversalement au travers de la compresse, le trou pour la cordelette de retrait étant situé plus près de l'extrémité de retrait que de l'extrémité d'insertion.

4. Tampon selon la revendication 1, 2 ou 3, dans lequel la longueur L de la tête est plus grande que le diamètre D de la compresse.

5. Tampon selon la revendication 2, comprenent, en outre, un matériau de couverture perméable (32)(112) appliqué sur au moins une partie de la compresse avant qu'elle soit soumise à une compression radiale, le matériau de couverture perméable recouvrant l'extrémité de retrait de la compresse et le matériau de couverture en excès au niveau de l'extrémité de retrait étant replié dans l'évidement.

6. Tampon selon la revendication 5, dans lequel le matériau de couverture perméable (112) est formé d'une enveloppe entourant complètement la compresse, ladite enveloppe ayant une paroi d'extrémité inférieure (114) qui est contiguë à l'extrémité d'insertion (115) de la compresse, une paroi tabulaire ( 116) qui entoure la surface périphérique de la compresse et une partie froncée (118) qui est repliée dans l'évidement (120) s'étendant dans la direction axiale depuis l'extrémité de retrait vers l'intérieur de la compresse.

7. Tampon selon l'une quelconque des revendications précédentes, dans lequel, avant la compression radiale, au moins certaines des couches de matériau absorbant déplacées sont déplacées d'une certaine distance sur la longueur de la compresse et amendées à faire saillie vers l'extérieur au niveau de l'extrémité d'insertion, pour donner une forme arrondie à l'extrémité d'insertion.

8. Tampon selon l'une quelconque des revendications précédentes, dans lequel le déplacement axial des parties centrales du matériau absorbant a pour effet d'augmenter la densité des fibres dans au moins certains endroits le long de la région centrale du tampon s'étendant dans la direction axiale par comparaison à la densité qui aurait été obtenue dans cette région s'il n'y avait pas eu ce déplacement.
